Europäisches Patentamt

European Patent Office

Office européen des brevets

(19)

(11) EP 0 583 354 B1

(12) EUROPEAN PATENT SPECIFICATION

(45) Date of publication and mention
of the grant of the patent:
**18.12.1996 Bulletin 1996/51**

(21) Application number: **92911194.6**

(22) Date of filing: **01.05.1992**

(51) Int Cl.[6]: **G01N 33/50**, C12Q 1/68

(86) International application number:
**PCT/US92/03649**

(87) International publication number:
**WO 92/19967 (12.11.1992 Gazette 1992/28)**

(54) **METHOD FOR OBTAINING INFORMATIVE CELLS**

VERFAHREN ZUR GEWINNUNG VON ZELLEN, DIE INFORMATIONEN TRAGEN

PROCEDE PERMETTANT D'OBTENIR DES CELLULES PORTEUSES D'INFORMATIONS

(84) Designated Contracting States:
**AT BE CH DE DK ES FR GB GR IT LI LU MC NL SE**

(30) Priority: **03.05.1991 US 695267**

(43) Date of publication of application:
**23.02.1994 Bulletin 1994/08**

(73) Proprietor: **IG LABORATORIES INC.**
**Framingham, MA 01701 (US)**

(72) Inventors:
• **KLINGER, Katherine, W.**
**Framingham, MA 01701 (US)**
• **PAVELKA, Karen**
**Framingham, MA 01701 (US)**
• **HOUSEAL, Timothy, W.**
**Hopkinton, MA 01748 (US)**
• **DACKOWSKI, William, R.**
**Hopkinton, MA 01748 (US)**

(74) Representative: **Deans, Michael John Percy**
**Lloyd Wise, Tregear & Co.,**
**Commonwealth House,**
**1-19 New Oxford Street**
**London WC1A 1LW (GB)**

(56) References cited:
**WO-A-90/10715**          **US-A- 4 888 278**

• **COLD SPRING HARBOR SYMPOSIA ON
QUANTITATIVE BIOLOGY vol. 32, 1968, COLD
SPRING HARBOR NY USA pages 349 - 351; E.P.
COHEN ET AL.: 'Antigen-unique species of RNA
in peritoneal cells of mice which do not adhere
to glass.'**
• **WO89/05337**
• **Develop. Biol., vol.61, p.114-117, (1977)**

## Description

In situ hybridization has been a major research tool for molecular geneticists to visualize specific DNA or RNA sequences present in cells. Originally performed in an isotopic format, non-isotopic techniques, such as fluorescence in situ hybridization (FISH), are rapidly becoming the method of choice, because they can be accomplished faster, multiple signals can be detected at one-time and hybridization signals can be more precisely located.

In general, however, non-isotopic methods have suffered from a lack of sensitivity, especially if the samples to be analyzed include dead and/or degrading cells. Dead or degrading cells appear to be resistant to in situ hybridization, possibly because the nuclear DNA itself has begun to degrade and/or because the cells do not respond to the chemical reagents used in processing. The presence within a sample of cells which are resistant to in situ hybridization makes the analysis of any informative cells, which may also be present, difficult.

US-A-4888 278 discloses a method of in situ hybridization of a probe to fixed cells. The cells are plated onto a solid substrate and cultured for 3 days on the substrate. WO89/05357 disclosed a method of in situ hybridization wherein CaSki cells are spotted on glass slides and grown for 12 hours at 37°C Developmental Biology 61 p 114-117 (1977) discloses a modified glass bead column for separating live from dead mouse spermatozoa

A method for preferentially isolating informative cells which produce clear hybridization signals in an in situ hybridization assay would be useful.

Summary of the Invention

In one aspect, the subject invention relates to a method for separating cells, which are informative in in-situ hybridization assays, from non-informative cells in a biological sample by: a) obtaining cellular material from a biological sample; b) transferring the material onto a substrate; and c) incubating the cellular material on the substrate for a period of time in the range of 1 to 60 minutes for the settling of informative cells and not non-informative cells onto the surface of the substrate.

In another aspect, the invention features methods for preparing cellular material for in situ hybridization using the method for separating informative cells from non-informative cells outlined above. Once deposited, informative cells are contacted with a fixative which preserves and retains nucleic acids within the cellular material. Fixed cellular material can then be analyzed using in situ hybridization techniques. In preferred embodiments, the cellular material is contacted with a protease inhibitor (e.g. phenylmethylsulfonylfluoride or benzamidine hydrochloride) and/or a salt of a short chain fatty acid (e.g. sodium butyrate, sodium propionate or sodium valerate) prior to fixation.

In a preferred method which is particularly useful for preparing cellular material obtained from a sample having few informative cells, cellular material is obtained from the sample and transferred onto a substrate which contains a protease inhibitors (e.g. phenylmethylsulfonylfluoride or benzamidine hydrochloride) and/or a salt of a short chain fatty acid (e.g. sodium butyrate, sodium proprionate or sodium valerate) Deposited cells can then be fixed in preparation for in situ analysis.

The methods of the subject invention result in clean preparations of informative cells having large and flat nuclei. In situ analysis of such preparations is easier to perform and results are more interpretable. Therefore, using the disclosed methods, diagnostic evaluations based on in situ analysis can be made with increased confidence.

Detailed Description of the Invention

One embodiment of the subject invention is based upon the surprising finding that informative cells can be separated from non-informative cells by a settling procedure. Informative cells are cells which provide an interpretable hybridization signal in an in situ hybridization assay. In contrast, non-informative cells, such as dead or degrading cells, do not provide an interpretable hybridization signal. Another embodiment of the invention is based on the finding that certain agents can be added during cell settling to produce clean preparations of informative cells having large and flat nuclei which can be more readily analyzed by in situ hybridization analysis.

Cellular material, such as fetal cells (e.g. obtained from amniotic fluid, maternal blood, cord blood, chorionic villus tissue or cervical secretions), lymphocytes (e.g. obtained from blood) embryonic cells (e.g. obtained from a biopsied embryo), and cancer cells (e.g. obtained from a tumor) can be obtained from a biological sample using procedures which are known in the art.

Cellular material can then be deposited on a substrate such as glass, plastic or nitrocellulose. A glass microscope slide is preferred, because it can be readily manipulated and viewed under a microscope. The substrate can be pretreated with a "cell adherent" which improves the likelihood that a cell settling onto the surface remains attached during subsequent manipulations. A preferred cell adherent is 3-Aminopropyltriethoxysilane. Treatment with this adherent results in "silanized" substrates. Other adherents useful in the subject invention include: poly-L lysine and mussel adhesin. Pretreatment of solid substrates can be accomplished using any method that ensures that cell adherent is deposited (e.g. submersion, transferring using a dropper, etc.). Pretreated solid substrates can be stored in a dust free environment at room temperature.

Once deposited onto a substrate, cellular material can be incubated to preferentially settle informative cells. The period of incubation must be long enough for a sufficient number of cells having intact cellular membranes to settle, but not so long that cellular membranes become damaged and cell and other debris settles. The appropriate incubation period can range from 1 to 60 minutes with from about 15 to about 30 minutes being optimal. There does not appear to be a strong dependence on temperature for preferential cell settling. For example, experiments have been conducted at room temperature and at 37° C with no clear difference in the quality of the preparation.

Certain agents can be added to the buffered solution during settling to further enhance the preferential deposition of informative cells. For example, phenylmethylsulfonylfluoride (PMSF) has been found to improve settling efficiency. It is reasonable to assume that other protease inhibitors such as benzamidine hydrochloride (BZA) will also prove useful for settling informative cells in preference to noninformative cells.

The settling procedure described above results in the separation of informative cells from noninformative cells. If the informative cells are then to be analyzed, for example in a hybridization assay, they can be treated further to produce clean preparations having large and flat nuclei. For example, a salt of a short chain fatty acid (such as sodium butyrate, sodium propionate and sodium valerate) when added to the preparation appears to free nuclei from cells, thereby rendering the nuclei more available for in situ hybridization. Also, incubation of informative cells in a hypotonic solution has been found to induce cells to swell, thereby spreading the cellular constituents and increasing the probability that the nucleus will be well-exposed in the final preparation.

Informative cells can then be processed through a fixation protocol to preserve the nuclei or chromosome in a morphologically stable state so that nucleic acids are retained through the rigorous conditions present during in situ hybridization. Appropriate fixatives are well-known in the art and include, for example, 4% paraformaldehyde or glutaraldehyde in phosphate buffered solution (PBS) containing 5mM $MgCl_2$, a fixative containing 3 parts ethanol and 1 part acetic acid, Carnoy's fixative, 1% osmium tetraoxide, Bouin's fixative, Zenker's fixative. In order to prevent loss of cellular material from a support, the fixative can either be applied directly to deposited (settled) material, in a small amount or a larger volume of a diluted solution can be applied. The supports can then be dried in preparation for in situ analysis.

A kit comprising the solutions of the subject invention which can be used for preparing cellular material can be produced. It can include, for example, a container for holding the required components, supports on which cellular material can be deposited, solutions for fixing informative cells, a protease inhibitor (e.g. PMSF or BZA) and a short chain fatty acid (e.g. sodium butyrate, sodium propionate or sodium valerate). A kit may also optionally include probes and solutions to stain probes.

The present invention will now be illustrated by the following examples, which are not intended to be limiting in any way.

Example 1: Method for Preparing a Cell Sample for In Situ Analysis

Solutions

PBS:
    32 g NaCl, 0.8 g KCl and 8.68 g NaH2PO4 7H2O. Make up to 4 liters with distilled H2O, pH to 7.5.
Carnoy's Fixative:
    75 ml methanol
    25 ml glacial acetic acid
0.075M KCl:
    Dissolve 5.59 g KCl in 1 liter distilled H2O. Filter sterilize using 0.2 μm filter flask.
30% Fix in 0.075M KCl:
    3 ml Carnoy's Fixative
    7 ml KCl

Preparation of Aminoalkylsilane Coated Slides

A 2% solution of 3-Aminopropyltriethoxysilane was prepared by mixing 5 mls of 3-Aminopropyltriethoxysilane with 250 ml acetone. Clean glass microscope slides were placed in a slide rack and submerged for 2 minutes in the 2% solution described above. Following submersion, slides were removed from solution and rinsed in two changes of distilled water. Slides were then drained and allowed to air dry. Prepared slides were stored in a dust-free box at room temperature.

<u>Sample Preparation for Settling Onto Slides</u>

An amniotic fluid sample was spun in a centrifuge tube at 2100 RPM (1029 G) for 7 minutes at room temperature. The supernatant was then removed by aspiration. The remaining pellet was resuspended in 50 μl of PBS per 1 ml of original fluid volume. 25 μl of resuspended pellet in PBS was then placed on a silanized glass microscope slide (prepared as described above). If a substantial amount of blood was present in the amniotic fluid, 1 drop of 30% 3:1 methanol:glacial acetic acid fixative (Carnoy's fixative) in 70% 0.075M KCl was added.

Slides were placed horizontally in a humid chamber at a temperature of 37 °C for 15 minutes. This incubation period allows informative cells to settle onto the surface of the silanized slide surface undisturbed while dead cells, or other cells having damaged membranes, remain floating in the buffer.

<u>Preparation of Cells Settled Onto Slides for In Situ Analysis</u>

After 15 minutes, 50μl of 0.075M KCl hypotonic solution (prewarmed to 37 °C was gently added to the buffered solution containing the resuspended cell pellet followed by an additional 15 minutes incubation in the heated humid chamber. Hypotonic solution causes viable cells to swell, making it easier to remove cytoplasm and cell membrane in subsequent steps.

After 15-minutes of incubating in KCl at 37 °C the fluid was gently tipped from each slide (to the side, not lengthwise). The slides were not allowed to dry at this step. As each slide was tipped off, it was placed flat on a paper towel and 100 μl of 30% Carnoy's fixative in 70% 0.075 M KCl was gently added. The slides were left undisturbed in this condition for a period of 5 minutes. The 30% fix/KCl solution ruptures the swollen cell membranes and conditions the cells to the fix.

After a 5 minute incubation period, fluid was gently tipped from each slide. The slides were not allowed to dry out. 5-6 drops of fresh undiluted Carnoy's fixative were immediately dropped onto the region of the slide containing the fetal cells. The slides were incubated for approximately 5 minutes then transferred to a 60 °C slide warmer and allowed to dry. This fixative treatment ruptures any remaining membranes and rinses away residual debris while fixing the nuclei to the slide.

Once dried, slides containing fixed cells were processed through an ethanol series prior to hybridization. In coplin jars, the slides were contacted sequentially with 70%, 80%, 90% and 100% ethanol for 1 minute in each dilution. The slides were then allowed to dry at room temperature.

<u>Example 2: Method for Preparing a Cell Sample for In Situ Analysis Which Includes the Addition of Sodium Butyrate and PMSF</u>

Amniotic fluid samples were prepared as described in Example 1, but with the following modifications:

1. Sodium butyrate was added to a concentration of 10mM and samples were incubated at room temperature for up to 2 hrs. Subsequent experiments have shown that this incubation may be as little as 5 minutes or in fact that incubation can be omitted entirely.

2. 20mM sodium butyrate and 0.5mM phenylmethylsulfonylfluoride were added to the buffer used to resuspend the pellet generated by centrifugation.

**In Situ Hybridization Analysis**

Fixed slides were placed on a 60°C slide warmer for 1-2 hours before hybridization. A nucleic acid probe from an endogenous mouse sequence about 35 kbs in length was labeled with biotin by nick translation. Each hybridization reaction contained 5-10 ng/μl of the biotin labelled probe, and 100 ng/μl of mouse Cot-1 DNA, 900 ng/μl of salmon DNA in a cocktail of 50% deionized formamide, 6XSSC, and 10% Dextran Sulphate. A 5-10μl aliquot of hybridization cocktail was applied to each site, coverslipped, and sealed with rubber cement. Probe and target were simultaneously denatured for 7-10 min at 80°C. After overnight hybridization at 37°C, slides were processed in the following manner: Slides were washed for 3 - 5 min in 50% formamide/2XSSC at 42°C, 1 - 5 min in 2XSSC at room temperature, and 3 - 5 min in 0.1XSCC at 60°C. Non-specific binding sites were blocked by incubation at 37°C in 3% BSA/4XSSC for 5 min. Hybridized probe was detected with a solution containing 2.0 ng/μl of Cy3 conjugated to streptavidin in 1% BSA, 0.1% Tween 20® and 4X SSC; preparations were incubated in this solution for 20 min at 37°C. Following detection, slides were washed for 3 - 5 min in 4 X SSC with 0.1% Tween 20® at 42°C, then washed in 2X SSC for at least 5 min at room temperature. Preparations were counterstained with 4'6-diamidino-2-phenylindole (DAPI) and mounted in 2.33% DABCO (Sigma, #D2522) in 100mM Tris (8.0) and 90% (vol:vol) glycerol. Post hybridization washing and detection are essentially as described in Klinger et. al. Am. J. Hum. Genet. 51(1): in press (1982).

**Results:**

Many more informative nuclei were recovered than when using the protocol described in Example 1 in a side-by-side comparison with the same amniotic fluid sample. Anywhere from 3-25 times more nuclei were recovered and, of those, 88% (on average) were able to hybridize. There was a greater absolute number of nuclei and more of these were informative. Therefore, less sample is required for performing an analysis. Most hybridization signals were in the same focal plane or could be seen from one focal plane, suggesting that these nuclei were also flatter than those recovered using the protocol in Example 1.

Example 3: In Situ Analysis of Cells From Embryos

**Embryo Retrieval and Cell Preparation for FISH**

Embryos at the 8-16 cell stage were flushed from the uteri/oviducts of pregnant females with Hanks Buffered Salt Solution (HBSS) and BSA. The zona pellucida was removed from the morula by a brief exposure to prewarmed acidic Tyrodes solution (Manipulating the Mouse Embryo: A Laboratory Manual ed. Hogan, Constantini and Lacy (Cold Spring Harbor Laboratory, 1986). The morula stage embryos were then incubated in calcium-free CZB medium with 5mg/ml BSA for 20 min. at 37°C in 5% $CO_2$ in air to decompact the embryos. The embryos were then disaggregated to single cells using a polished micropipet. The dissociated morula cells were then processed for FISH.

The cells were transferred into 10μl of TB buffer (T Buffer: 1mM Tris; 25mM KCl; 0.9mM $CaCl_2$; and 0.9 mM$MgCl_2$ at pH 7.6 ; TB Buffer: T Buffer plus 20mM sodium butyrate or TB/PMSF buffer: TB buffer containing 1/100 dilution of 50mM PMSF in isopropanol) on a siliconized Teflon$^{Tm}$ masked glass slide (Cel-line Assoc, Newfield, N.J.) for 15 min. Then 20 μl of 0.3% hypotonic sodium chloride was added to the drop for 15 min. Fixation was achieved by adding 5 μl of Carnoy's (3:1 methanol:acetic acid solution) for 10 min, followed by the addition of 40-60 μl of Carnoy's. The samples were allowed to air dry or in some cases, the excess fixative was removed by tipping the slide briefly on its side, then allowed to dry slowly in a humid chamber.

In Situ Hybridization Analysis

Fixed slides were placed on a 60°C slide warmer for 1-2 hours before hybridization. A nucleic acid probe from an endogenous mouse sequence about 35 kbs in length was labelled with biotin by nick translation. Each hybridization reaction contained 5-10 ng/μl of the biotin labelled probe, and 100 ng/μl of mouse Cot-1 DNA, 900 ng/μl of salmon DNA in a cocktail of 50% deionized formamide, 6XSSC, and 10% Dextran Sulphate. A 5-10μl aliquot of hybridization cocktail was applied to each site, coverslipped, and sealed with rubber cement. Probe and target were simultaneously denatured for 7-10 min at 80°C. After overnight hybridization at 37°C, slides were processed in the following manner: Slides were washed for 3-5 min in 50% formamide/2XSSC at 42°C, 1-5 min in 2XSSC at room temperature, and 3-5 min in 0.1XSCC at 60°C. Non-specific binding sites were blocked by incubation at 37°C in 3% BSA/4XSSC for 5 min. Hybridization probe was detected with a solution containing 2.0 ng/μl of Cy3 conjugated to streptavidin in 1% BSA, 0.1% Tween 20® and 4 X SSC; preparations were incubated in this solution for 20 min at 37°C. Following detection, slides were washed for 3 - 5 min in 4 X SSC with 0.1% Tween 20® at 42°C, then washed in 2 X SSC for at least 5 min at room temperature. Preparations were counterstained with 4',6-diamidino-2-phenylindole (DAPI) and mounted in 2.33% DABCO (Sigma, #D2522) in 100mM Tris (8.0) and 90% (vol:vol) glycerol. Post hybridization washing and detection are essentially as described in Klinger et. al. Am. J. Hum. Genet. 51(1): in press (1982).

**Results**

The results are summarized in Table 1. A total of 209 morula cells were obtained from three independent experiments; 166 of these (79.5%) were recovered after processing in the manner described. Hybridization signal was detected in all (100%) of the recovered cells. These results demonstrate good recovery of embryonic cells (nearly 80%) and 100% hybridization efficiency. These results suggest that these procedures can be applied to the analysis of biopsied cells from preimplantation embryo.

TABLE 1

| Experiment | Cell Recovery (%) | Hybridization Signal |
|------------|-------------------|----------------------|
| 1 | 55 of 78 (71%) | 55 |

TABLE 1   (continued)

| Experiment | Cell Recovery (%) | Hybridization Signal |
|------------|-------------------|----------------------|
| 2 | 57 of 77 (74%) | 57 |
| 3 | 54 of 54 (100%) | 54 |
| TOTAL | 166 of 209 (79.5%) | 166 |

**Claims**

1. A method for separating cells, which are informative in in-situ hybridization assays, from non-informative cells present in a biological sample, comprising the steps of:

   a) obtaining cellular material from a biological sample;
   b) transferring the cellular material obtained in step a) to a substrate; and
   c) incubating the cellular material on the substrate for

   a period of time in the range of 1 to 60 minutes for the settling of informative cells and not non-informative cells onto the surface of the substrate.

2. A method according to Claim 1, wherein the substrate is pretreated with a cell adherent.

3. A method according to Claim 1, wherein the cellular material is contacted with a protease inhibitor.

4. A method according to Claim 3, wherein the protease inhibitor is selected from the group consisting of phenylmethylsulfonylfluoride and benzamidine hydrochloride.

5. A method according to Claim 4, wherein the protease inhibitor is phenylmethylsulfonylfluoride.

6. A method according to Claim 1, wherein the cellular material is contacted with a salt of a short chain fatty acid.

7. A method according to Claim 6, wherein the salt of the short chain fatty acid is selected from the group consisting of sodium butyrate, sodium propionate and sodium valerate

8. A method according to Claim 7, wherein the salt of the short chain fatty acid is sodium butyrate.

9. A method according to Claim 1, wherein said cells are fetal cells.

10. A method according to Claim 1, wherein the biological sample is selected from the group consisting of amniotic fluid, chorionic villus tissue, maternal cervical secretions, cord blood and maternal blood.

11. A method for preparing cellular material for in situ hybridization analysis, comprising the steps of:

   separating informative cells from non-informative cells by the method according to any of Claims 1 to 8 or 10; and contacting the cellular material with a fixative, thereby producing fixed cellular material.

12. A method according to Claim 11, wherein the biological sample is an embryo.

**Patentansprüche**

1. Verfahren zur Trennung von Zellen, die in einem in-situ-Hybridisierungsassay Information tragen, von nicht-informationstragenden Zellen, die in einer biologischen Probe vorhanden sind, umfassend die Stufen:

   a) Gewinnen eines zellulären Materials aus einer biologischen Probe;
   b) Übertragen des in Stufe a) erhaltenen zellulären Materials auf ein Substrat; und
   c) Inkubieren des zellulären Materials auf dem Substrat für eine Zeitspanne im Bereich von 1 bis 60 Minuten, wobei sich informationstragende Zellen auf der Oberfläche des Substrats ansiedeln und die nicht-informationstragenden Zellen nicht.

2. Verfahren nach Anspruch 1, dadurch **gekennzeichnet**, daß das Substrat mit einem Zellhaftmittel vorbehandelt wird.

3. Verfahren nach Anspruch 1, dadurch **gekennzeichnet**, daß das zelluläre Material mit einem Protease-Inhibitor in Kontakt gebracht wird.

4. Verfahren nach Anspruch 3, dadurch **gekennzeichnet**, daß der Protease-Inhibitor ausgewählt wird aus der Gruppe, bestehend aus Phenylmethylsulfonylfluorid und Benzamidinhydrochlorid.

5. Verfahren nach Anspruch 4, dadurch **gekennzeichnet**, daß der Protease-Inhibitor Phenylmethylsufonylfluorid ist.

6. Verfahren nach Anspruch 1, dadurch **gekennzeichnet**, daß das zelluläre Material mit einem Salz einer kurzkettigen Fettsäure in Kontakt gebracht wird.

7. Verfahren nach Anspruch 6, dadurch **gekennzeichnet**, daß das Salz der kurzkettigen Fettsäure ausgewählt wird aus der Gruppe, bestehend aus Natriumbutyrat, Natriumpropionat und Natriumvalerat.

8. Verfahren nach Anspruch 7, dadurch **gekennzeichnet**, daß das Salz der kurzkettigen Fettsäure Natriumbutyrat ist.

9. Verfahren nach Anspruch 1, dadurch **gekennzeichnet**, daß die Zellen fetale Zellen sind.

10. Verfahren nach Anspruch 1, dadurch **gekennzeichnet**, daß die biologische Probe ausgewählt wird aus der Gruppe, bestehend aus Amnionflüssigkeit, Chorionzottengewebe, materne cervicale Sekrete, Nabelschnurblut und maternes Blut.

11. Verfahren zur Gewinnung des zellulären Materials für die in-situ-Hybridisierungsanalyse, umfassend die Stufen:

Trennung informationstragender Zellen von nicht-informationstragender Zellen nach einem Verfahren, gemäß den Ansprüchen 1 bis 8 oder 10;
und Inkontaktbringen des zellulären Materials mit einem Fixiermittel, wodurch ein fixiertes zelluläres Material hergestellt wird.

12. Verfahren nach Anspruch 11, dadurch **gekennzeichnet**, daß die biologische Probe ein Embryo ist.


**Revendications**

1. Procédé pour séparer des cellules qui sont porteuses d'informations dans des tests d'hybridation in situ d'avec des cellules non porteuses d'informations présentes dans un échantillon biologique, comprenant les étapes :

a) d'obtention de matériel cellulaire à partir d'un échantillon biologique ;
b) de transfert du matériel cellulaire obtenu dans l'étape a) à un substrat ; et
c) d'incubation du matériel cellulaire sur le substrat pendant une durée située dans le domaine de 1 à 60 min pour la sédimentation de cellules porteuses d'informations et non de cellules non porteuses d'informations sur la surface du substrat.

2. Procédé selon la revendication 1, dans lequel le substrat est prétraité avec un adhésif pour cellules.

3. Procédé selon la revendication 1, dans lequel le matériel cellulaire est mis en contact avec un inhibiteur de protéase.

4. Procédé selon la revendication 3, dans lequel l'inhibiteur de protéase est choisi dans le groupe consistant en le fluorure de phénylméthylsulfonyle et le chlorhydrate de benzamidine.

5. Procédé selon la revendication 4, dans lequel l'inhibiteur de protéase est le fluorure de phénylméthylsulfonyle.

6. Procédé selon la revendication 1, dans lequel le matériel cellulaire est mis en contact avec un sel d'un acide gras à chaîne courte.

7. Procédé selon la revendication 6, dans lequel le sel de l'acide gras à chaîne courte est choisi dans le groupe consistant en le butyrate de sodium, le propionate de sodium et le valérate de sodium.

8. Procédé selon la revendication 7, dans lequel le sel de l'acide gras à chaîne courte est le butyrate de sodium.

9. Procédé selon la revendication 1, dans lequel lesdites cellules sont des cellules foetales.

10. Procédé selon la revendication 1, dans lequel l'échantillon biologique est choisi dans le groupe consistant en le liquide amniotique, le tissu de villosités choriales, les sécrétions cervicales maternelles, le sang du cordon et le sang maternel.

11. Procédé pour préparer un matériel cellulaire pour analyse par hybridation in situ, comprenant les étapes :

de séparation des cellules porteuses d'informations d'avec les cellules non porteuses d'informations par le procédé selon l'une quelconque des revendications 1 à 8 ou 10 ;
et de mise en contact du matériel cellulaire avec un fixateur pour produire un matériel cellulaire fixé.

12. Procédé selon la revendication 11, dans lequel l'échantillon biologique est un embryon.